# EUROPEAN PATENT APPLICATION

(11) **EP 4 183 879 A1**
(43) Date of publication of application: **24.05.2023**
(21) Application number: 21809288.0
(22) Date of filing: 21.05.2021
(51) Int. Cl.: C12N 15/113, A61K 31/713, A61K 48/00, A61K 9/14, A61K 9/19, A61P 31/14

(54) **DOUBLE-STRANDED OLIGONUCLEOTIDE AND COMPOSITION FOR TREATING COVID-19 CONTAINING SAME**

(30) Priority: 22.05.2020 KR 20200061828
(71) Applicant: Bioneer Corporation, Daejeon 34302 (KR); Sirnagen Therapeutics Corporation, Daejeon 34302 (KR)
(72) Inventor: PARK, Han-Oh, Sejong-si 30151 (KR); KIM, Jangseon, Daejeon 34011 (KR); LEE, Mi Sun, Daejeon 34011 (KR); CHOI, Soonja, Daejeon 34048 (KR); LEE, Eun-Kwang, Daejeon 34310 (KR); BYUN, Sang Jin, Daejeon 35265 (KR)
(74) Representative: Mewburn Ellis LLP
(86) International application number: PCT/IB2021/054404
(87) International publication number: WO 2021/234647

(57) **Abstract**

The present invention relates to: a double-stranded oligonucleotide which can highly specifically and efficiently inhibit the proliferation of Severe acute respiratory syndrome coronavirus 2 (SARS-Cov-2), preferably a double-stranded oligonucleotide comprising a sequence in the form of RNA/RNA, DNA/DNA or a DNA/RNA hybrid; a double-stranded oligonucleotide structure and nanoparticles comprising the double-stranded oligonucleotide; and a use thereof for treating COVID-19.

## Description

### [Technical Field]

The present invention relates to a double-stranded oligonucleotide capable of very specifically inhibiting the proliferation of SARS-Cov-2 (severe acute respiratory syndrome coronavirus 2) with high efficiency, preferably a double-stranded oligonucleotide including a sequence in the form of RNA/RNA, DNA/DNA, or a DNA/RNA hybrid, a double-stranded oligonucleotide construct including the double-stranded oligonucleotide, nanoparticles including the same, and the use thereof for the treatment of COVID-19.

### [Background Art]

In 1995, Guo and Kemphues discovered that sense RNA was as effective as antisense RNA in inhibiting gene expression using antisense in C. *elegans,* so research has been conducted to determine the cause thereof. In 1998, Fire et al. first found a phenomenon by which double-stranded RNA (dsRNA) is injected and mRNA corresponding thereto is specifically degraded to thus inhibit gene expression, which is termed RNA interference (RNAi). RNAi, which is a method used to inhibit gene expression, is capable of clearly showing the effect of inhibition of gene expression at a low cost in a simple manner, and thus the range of application thereof is increasing.

Because technology for inhibiting gene expression is able to regulate the expression of specific genes, target genes in cancer, genetic diseases, and the like, which are caused by overexpression of specific genes, may be eliminated at the mRNA level, based on which this technology may be used as an important tool for the development of therapeutic agents for disease treatment and verification of targets. Conventionally, in order to inhibit the expression of a target gene, technology for introducing a transgene to the target gene is disclosed, and examples thereof include a method of introducing a transgene in the reverse direction (antisense) of the promoter and a method of introducing a transgene in the forward direction (sense) of the promoter.

RNA therapy targeting RNA is a method that removes the function of the corresponding gene using oligonucleotides for target RNA, and is said to be different from conventional therapeutics, such as those using antibodies and small molecules, which mainly target proteins. There are two major approaches to targeting RNA: double-stranded-RNA-mediated RNAi and use of an antisense oligonucleotide (ASO). Currently, clinical trials to target RNA are being attempted for various diseases.

An antisense oligonucleotide (hereinafter referred to as 'ASO') is short synthetic DNA designed to bind to a target gene based on Watson-Crick base pairing, and has been used to study gene function and to develop therapeutics capable of treating diseases such as cancer at the molecular level because it is capable of specifically inhibiting the expression of a certain nucleotide sequence of a gene. ASO has an advantage in that it may be easily produced by variously setting the inhibition of gene expression, and there have been studies on the use of the same to inhibit the expression of oncogenic genes and the growth of cancer cells. The process by which ASO inhibits the expression of a specific gene is achieved by eliminating mRNA due to RNase H activity induced by binding to a complementary mRNA sequence or by interfering with the formation and progression of a ribosome complex for protein translation. It has also been reported that ASO binds to genomic DNA to form a triple-helix structure, thereby inhibiting gene transcription. Although ASO has the above potential, in order to use the same in clinical practice, it is necessary to improve resistance to nucleases and to be efficiently delivered into target tissues or cells so as to specifically bind to the nucleotide sequence of a gene of interest. Moreover, the secondary and tertiary structures of gene mRNA are important for specific binding of ASO, and since a region in which less of the secondary structure of mRNA is formed is very advantageous for ASO to access, efforts have been made to effectively achieve gene-specific inhibition *in vitro* as well as *in vivo* by systematically analyzing a region in which less of the secondary structure of mRNA is formed before ASO synthesis. This ASO is more stable than siRNA, which is a kind of RNA, and has the advantage of being easily soluble in water and saline. Currently, three ASOs have been approved by the Federal Drug Administration (FDA) (Jessica, C., J. Postdoc. Res., 4:35-50, 2016).

Since discovery of the roles thereof, RNA interference (hereinafter referred to as 'RNAi') has been found to act on sequence-specific mRNA in various types of mammalian cells (Barik, S., J. Mol. Med. (2005) 83: 764-773). When long-chain double-stranded RNA is delivered to cells, the delivered double-stranded RNA is processed into small interfering RNA (hereinafter referred to as 'siRNA') of 21 to 23 base pairs (bp) in length by an endonuclease called a dicer, and siRNA binds to RISC (RNA-induced silencing complex) to thus inhibit the expression of a target gene in a sequence-specific manner through a process in which the guide (antisense) strand recognizes and degrades target mRNA. Gene expression inhibition technology using siRNA is useful in research to identify the function of the target gene in the target cell by inhibiting the expression of the target gene in the target cell and observing changes due thereto. In particular, inhibiting the function of a target gene in an infectious virus or cancer cell will be useful in developing a treatment method for the disease, and it has been reported that siRNA is able to inhibit the expression of target genes based on the results of *in-vitro* studies and *in-vivo* studies using experimental animals.

According to Bertrand's research team, it has been found that siRNA for the same target gene has a strong inhibitory effect on the expression of mRNA *in vitro* and *in vivo* compared to an antisense oligonucleotide (ASO), and that this effect lasts for a long time. Moreover, since the mechanism of action of siRNA is to regulate the expression of a target gene in a sequence-specific manner by complementary binding to target mRNA, it is advantageous in that the range of targets to which siRNA is applicable is dramatically expanded compared to conventional antibody-based drugs or small molecule drugs (M.A. Behlke, MOLECULAR THERAPY. 2006 13(4):664-670).

Despite the excellent effect of siRNA and the wide range of use thereof, for siRNA to be developed as a therapeutic agent, siRNA must be effectively delivered to target cells by improving *in-vivo* stability of siRNA and increasing cell delivery efficiency (F.Y. Xie, Drug Discov. Today, 2006 Jan.; 11(1-2):67-73). In order to improve *in-vivo* stability and solve problems related to nonspecific innate immune stimulation of siRNA, thorough research into modification of some nucleotides or backbones of siRNA so as to be imparted with nuclease resistance, use of a carrier such as a viral vector, liposome or nanoparticles, etc. is ongoing.

A delivery system using a viral vector such as an adenovirus or a retrovirus has high transfection efficacy, but immunogenicity and oncogenicity are high. On the other hand, a non-viral delivery system including nanoparticles has lower cell delivery efficiency than a viral delivery system, but is advantageous because *in-vivo* stability is high, target-specific delivery is possible, RNAi oligonucleotides are taken up by and internalized into cells or tissues, and there is almost no cytotoxicity or immune stimulation, so the non-viral delivery system is currently considered to be a powerful delivery method compared to the viral delivery system (Akhtar S., J. Clin. Invest. 2007 December 3; 117(12): 3623-3632).

The method of use of a nanocarrier among non-viral delivery systems includes forming nanoparticles using various polymers such as liposomes, cationic polymer complexes, and the like and loading siRNA on such nanoparticles, namely nanocarriers, to thus deliver the same to cells. Here, specific examples of the nanocarrier may include polymeric nanoparticles, polymer micelles, lipoplexes, and the like. In particular, the lipoplex, which is composed of cationic lipids, interacts with the anionic lipids of the cellular endosome to induce a destabilizing effect of the endosome, thereby realizing intracellular delivery.

In addition, it is known that it is possible to induce high efficiency *in vivo* by linking a chemical to the end portion of an siRNA passenger (sense) strand to impart enhanced pharmacokinetics (J. Soutschek, Nature 11; 432(7014):173-8, 2004). Here, the stability of siRNA varies depending on the properties of the chemical bound to the end of the siRNA sense (passenger) or antisense (guide) strand. For example, siRNA in a form in which a polymer compound such as polyethylene glycol (PEG) is conjugated thereto interacts with the anionic phosphate group of siRNA in the presence of a cationic material to form a complex, thereby obtaining a delivery carrier having improved siRNA stability (S.H. Kim, J. Control Release 129(2):107-16, 2008). In particular, micelles composed of polymer complexes are extremely small in size compared to other systems used as drug delivery carriers, such as microspheres or nanoparticles, but the distribution thereof is very uniform and occurs spontaneously, resulting in easy formulation quality control and reproducibility.

In order to improve the intracellular delivery efficiency of siRNA, technology has been developed for realizing efficient cell membrane permeability and stability of siRNA using an siRNA conjugate in which a biocompatible polymer hydrophilic material (e.g. polyethylene glycol (PEG)) is conjugated to siRNA through a simple covalent bond or a linker-mediated covalent bond (Korean Patent No. 883,471). However, even when siRNA is chemically modified or conjugated to polyethylene glycol (PEG) (PEGylation), it still has disadvantages such as low *in-vivo* stability and poor delivery to target organs. In order to overcome these disadvantages, a double-stranded oligo RNA construct in which hydrophilic and hydrophobic materials are bound to an oligonucleotide, particularly double-stranded oligo RNA such as siRNA, has been developed, and the construct forms self-assembled nanoparticles called SAMiRNA^{™} (self-assembled micelle inhibitory RNA) through hydrophobic interaction of the hydrophobic material (Korean Patent No. 1,224,828). Here, SAMiRNA^{™} technology is capable of realizing homogenous nanoparticles having a very small size compared to conventional delivery methods.

In a specific embodiment of SAMiRNA^{™} technology, a hydrophilic material such as PEG (polyethylene glycol) or HEG (hexaethylene glycol) is used. PEG is a synthetic polymer and is often used to increase the solubility of pharmaceuticals, particularly proteins, and to control pharmacokinetics. PEG is a polydisperse material, and a polymer in one batch is composed of a sum of different numbers of monomers, so the molecular weight thereof shows a Gaussian curve, and a polydispersity value (Mw/Mn) represents the extent of homogeneity of a material. Specifically, PEG, having a low molecular weight (3 to 5 kDa), has a polydispersity value of about 1.01, whereas PEG, having a high molecular weight (20 kDa), has a high polydispersity value of about 1.2. The higher the molecular weight, the lower the homogeneity of the material. Therefore, when PEG is conjugated to pharmaceuticals, the polydispersity of PEG is reflected in the conjugate, making it difficult to verify a single material, which is undesirable. Hence, a material having a low polydispersity value intends to be produced through improvement in PEG synthesis and purification processes. However, in particular, when PEG is bound to a material having a low molecular weight, there are problems related to the polydispersity of the material, such as inconvenience that it is not easy to determine whether binding is easily performed (Francesco M. V. DRUG DISCOVERY TODAY(2005) 10(21):1451-1458).

Accordingly, in recent years, as an improved form of conventional self-assembled nanoparticle SAMiRNA^{™} technology, blocks in which the hydrophilic material of the double-stranded oligonucleotide construct constituting SAMiRNA^{™} is blocked into a basic unit including 1 to 15 uniform monomers having a predetermined molecular weight and a linker as necessary are used in an appropriate number depending on the need, whereby a new type of delivery carrier technology with a small size and remarkably improved polydispersity compared to conventional SAMiRNA^{™} has been developed. It is already known that siRNA is rapidly degraded by various enzymes present in the blood when injected *in vivo,* thus resulting in poor delivery efficiency to target cells or tissues, and the improved SAMiRNA^{™} also shows that variation in stability and expression inhibition efficiency is observed depending on the target gene. Therefore, the present inventors have ascertained that the stability and expression inhibitory effect of the target gene may be remarkably enhanced by applying a double-stranded oligonucleotide in a DNA/RNA hybrid form using a DNA sequence that is an ASO sense strand as a guide and an RNA sequence that is an antisense strand as a passenger, in order to more stably and effectively inhibit the expression of a target gene using improved self-assembled nanoparticles SAMiRNA^{™}.

Meanwhile, COVID-19 is a respiratory syndrome caused by SARS-Cov-2 (severe acute respiratory syndrome coronavirus 2) infection. After an incubation period of about 2 to 14 days, the main symptoms of COVID-19 are fever, respiratory symptoms such as cough or shortness of breath, and pneumonia, and COVID-19 is known to spread due to droplets (saliva droplets) generated by coughing or sneezing or when people touch objects contaminated with SARS-Cov-2 and then touch their eyes, nose, and mouth.

Since COVID-19 first occurred in Wuhan, China in December 2019, it has spread throughout China and around the world. As of April 2020, there have been 2.8 million confirmed cases and more than 200,000 deaths. The World Health Organization (WHO) declared a Public Health Emergency of International Concern (PHEIC) on January 30, 2020 for COVID-19, and then on March 11, declared a pandemic for COVID-19. A pandemic, which is the highest level of infectious disease risk, means "the global spread of a virus for which the majority of people do not have immunity," and the number of confirmed cases, deaths, and affected countries continues to increase.

Coronavirus has a crown-shaped spike (S) protein that protrudes from the outer surface thereof under an electron microscope, and is a positive single-stranded (+ssRNA) virus that is circular or oval. Coronaviruses are classified into four genera: alphaCoV, betaCoV, deltaCoV, and gammaCoV. Examples of alphaCoV, which causes common respiratory infections, may include HCoV-229E and HCoV-NL63, and examples of betaCoV may include HCoV-OC43 and HCoV-HKU1, which may also lead to upper respiratory infections such as colds and digestive disorders. SARS-CoV (B lineage) and MERS-CoV (C lineage) belonging to betaCoV cause serious respiratory infectious diseases characterized by acute respiratory symptoms. The genomic sequence isolated from pneumonia patients in the early stages of COVID-19 was analyzed to have 89% nucleotide homology with bat SARS-like-CoVZXC21 and 82% homology with SARS-CoV, and this virus was found to belong to the betaCoV genus and was named SARS-CoV-2.

In Korea, in the early stages of the outbreak of COVID-19, a confirmatory test was conducted using a pan-coronavirus test (conventional PCR) and sequencing match. Currently, however, a real-time RT-PCR (reverse transcription polymerase chain reaction) test is being performed to diagnose COVID-19. Vaccine development has been completed by a number of pharmaceutical companies around the world and vaccination is in progress, but no effective therapeutic agents for COVID-19 have been released yet.

As mentioned above, technical development of RNAi therapeutic agents for COVID-19 and delivery systems therefor is still insignificant, and the market demand for a double-stranded oligonucleotide therapeutic agent capable of specifically inhibiting the proliferation of SARS-Cov-2 with high efficiency and delivery technology therefor is very high.

Accordingly, the present inventors selected a double-stranded oligonucleotide targeting SARS-Cov-2, and particularly identified an RNAi therapeutic agent capable of efficiently treating COVID-19 by inhibiting the proliferation of SARS-Cov-2 and a delivery carrier thereof, thus culminating in the present invention.

### [Citation List]

### [Patent Literature]

U.S. Patent No. 5,034,323
U.S. Patent No. 5,231,020
U.S. Patent No. 5,283,184
Korean Patent No. 883471
Korean Patent No. 1224828

### [Non-Patent Literature]

Jessica, C., J. Postdoc. Res., 4:35-50, 2016
Barik, S. J. Mol. Med. (2005) 83: 764-773
M.A. Behlke, MOLECULAR THERAPY. 2006 13(4):664-670
F.Y. Xie, Drug Discov. Today 2006 Jan.; 11(1-2):67-73
Akhtar S., J. Clin. Invest. 2007 December 3; 117(12): 3623-3632
J. Soutschek, Nature 11; 432(7014):173-8, 2004
S.H. Kim, J. Control Release 129(2):107-16, 2008
Francesco M. V. DRUG DISCOVERY TODAY(2005) 10(21):1451-1458
Moon Seong-sil, BRIC View 2020-TX2(2020)

### [Disclosure]

### [Technical Problem]

It is an object of the present invention to provide a double-stranded oligonucleotide capable of very specifically inhibiting the proliferation of SARS-Cov-2 (severe acute respiratory syndrome coronavirus 2) with high efficiency, preferably a double-stranded oligonucleotide including a sequence in the form of RNA/RNA, DNA/DNA, or a DNA/RNA hybrid, a double-stranded oligonucleotide construct including the double-stranded oligonucleotide, and nanoparticles including the same.

It is another object of the present invention to provide a pharmaceutical composition for treating COVID-19 containing, as an active ingredient, the double-stranded oligonucleotide, the double-stranded oligonucleotide construct including the double-stranded oligonucleotide, and/or the nanoparticles including the same.

It is still another object of the present invention to provide a method of treating COVID-19 including administering the pharmaceutical composition to a subject in need of treatment for COVID-19.

It is yet another object of the present invention to provide the use of the double-stranded oligonucleotide, the double-stranded oligonucleotide construct including the double-stranded oligonucleotide, and/or the nanoparticles including the same for the treatment of COVID-19.

It is still yet another object of the present invention to provide the use of the pharmaceutical composition for the treatment of COVID-19.

It is a further object of the present invention to provide the use of the double-stranded oligonucleotide, the double-stranded oligonucleotide construct including the double-stranded oligonucleotide, and/or the nanoparticles including the same for the manufacture of a medicament for the treatment of COVID-19.

### [Technical Solution]

In order to accomplish the above objects, the present invention provides a double-stranded oligonucleotide including a sense strand including any one sequence selected from the group consisting of SEQ ID NOs: 1, 2, 3, 4, 5, 6, 7, 8, 9, and 10 and an antisense strand including a sequence complementary thereto.

In addition, the present invention provides a double-stranded oligonucleotide construct including the double-stranded oligonucleotide and/or nanoparticles including the same.

In addition, the present invention provides a pharmaceutical composition for treating COVID-19 including a double-stranded oligonucleotide including a sense strand including any one sequence selected from the group consisting of SEQ ID NOs: 1, 2, 3, 4, 5, 6, 7, 8, 9, and 10 and an antisense strand including a sequence complementary thereto, a double-stranded oligonucleotide construct including the double-stranded oligonucleotide, or nanoparticles including the same.

In addition, the present invention provides a method of treating COVID-19 including administering the pharmaceutical composition to a subject in need of treatment for COVID-19.

A double-stranded oligonucleotide according to an aspect of the present invention is understood to include any material having general RNAi (RNA interference) action, and the double-stranded oligonucleotide specific to SARS-Cov-2 also includes SARS-Cov-2-specific shRNA, etc., as will be apparent to those skilled in the art to which the invention belongs. Simply put, the oligonucleotide may be siRNA, shRNA, or miRNA.

In the present invention, each of the sense strand and the antisense strand may independently be DNA or RNA, and a hybrid form in which the sense strand is DNA and the antisense strand is RNA or in which the sense strand is RNA and the antisense strand is DNA may be used.

In the present invention, SEQ ID NOs: 1 to 10 are described in the form of DNA, but when RNA is used, the sequences of SEQ ID NOs: 1 to 10 may be provided in the corresponding RNA sequence form in which T is changed to U.

Also, the double-stranded oligonucleotide according to the present invention includes not only a perfect match in which the sense strand of the sequence is a nucleotide sequence 100% complementary to the binding site of the SARS-Cov-2 gene, but also a mismatch in which some nucleotide sequences do not match, so long as specificity to SARS-Cov-2 is maintained.

The double-stranded oligonucleotide according to the present invention may include an overhang structure in which one or more unpaired nucleotides are provided at the 3' end of one or both strands.

In the present invention, the sense strand or the antisense strand is preferably composed of 19 to 31 nucleotides, but the present invention is not limited thereto.

In the present invention, the sense strand or the antisense strand of the double-stranded oligonucleotide may include any chemical modification in order to improve in-vivo stability or confer nuclease resistance and reduce nonspecific immune responses. The chemical modification may include, but is not limited to, at least one selected from the group consisting of a modification in which the hydroxyl group (-OH) at the 2' carbon position of the sugar structure in the nucleotide is substituted with any one selected from the group consisting of methyl (-CH₃), methoxy (-OCH₃), amine (-NH₂), fluorine (-F), O-2-methoxyethyl, O-propyl, O-2-methylthioethyl, O-3-aminopropyl, O-3-dimethylaminopropyl, O-N-methylacetamido, and O-dimethylamidooxyethyl; a modification in which oxygen of the sugar structure in the nucleotide is substituted with sulfur; a modification in which the nucleotide bond is any one bond selected from the group consisting of a phosphorothioate bond, a boranophosphate bond, and a methyl phosphonate bond; a modification into PNA (peptide nucleic acid), LNA (locked nucleic acid), or UNA (unlocked nucleic acid); and a modification in a DNA-RNA hybrid form (Ann. Rev. Med. 55, 61-65 2004; US 5,660,985; US 5,958,691; US 6,531,584; US 5,808,023; US 6,326,358; US 6,175,001; Bioorg. Med. Chem. Lett. 14:1139-1143, 2003; RNA, 9:1034-1048, 2003; Nucleic Acid Res. 31:589-595, 2003; Nucleic Acids Research, 38(17) 5761-773, 2010; Nucleic Acids Research, 39(5):1823-1832, 2011).

In the present invention, at least one phosphate group, preferably 1 to 3 phosphate groups, may be bound to the 5' end of the antisense strand of the double-stranded oligonucleotide.

Another aspect of the present invention pertains to a double-stranded oligonucleotide construct having the structure of Structural Formula (1) below, in which A is a hydrophilic material, B is a hydrophobic material, X and Y are each independently a simple covalent bond or a linker-mediated covalent bond, and R is a double-stranded oligonucleotide.

In a preferred embodiment, the double-stranded oligonucleotide construct including the SARS-Cov-2-specific sequence according to the present invention has the structure of Structural Formula (1) below.

Structural Formula (1) A-X-R-Y-B

In Structural Formula (1), A is a hydrophilic material, B is a hydrophobic material, X and Y are each independently a simple covalent bond or a linker-mediated covalent bond, and R is a SARS-Cov-2-specific double-stranded oligonucleotide.

The double-stranded oligonucleotide according to the present invention is preferably in the form of a DNA-RNA hybrid, siRNA (short interfering RNA), shRNA (short hairpin RNA), or miRNA (microRNA), but is not limited thereto, and also includes a single-stranded miRNA inhibitor that serves as an antagonist to miRNA.

Hereinafter, the double-stranded oligonucleotide according to the present invention will be described based on RNA, but may be applied to other double-stranded oligonucleotides having the same properties as the double-stranded oligonucleotide of the present invention, as will be apparent to those skilled in the art

More preferably, the double-stranded oligonucleotide construct including the SARS-Cov-2-specific double-stranded oligonucleotide according to the present invention has the structure of Structural Formula (2) below.

Structural Formula (2) A-X-S-Y-B AS

In Structural Formula (2), A, B, X, and Y are as defined in Structural Formula (1), S represents the sense strand of the SARS-Cov-2-specific nucleotide sequence, and AS represents the antisense strand of the SARS-Cov-2-specific double-stranded oligonucleotide.

More preferably, the double-stranded oligonucleotide construct including the SARS-Cov-2-specific double-stranded oligonucleotide has the structure of Structural Formula (3) or (4) below.

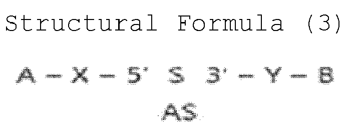

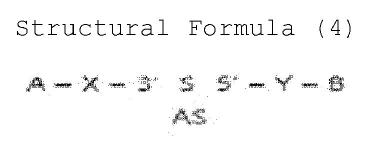

In Structural Formula (3) and Structural Formula (4), A, B, S, AS, X, and Y are as defined in Structural Formula (2), and 5' and 3' respectively represent the 5' end and the 3' end of the sense strand of the SARS-Cov-2-specific double-stranded oligonucleotide.

The hydrophilic material may be selected from the group consisting of polyethylene glycol (PEG), polyvinylpyrrolidone, and polyoxazoline, but is not limited thereto.

In the double-stranded oligonucleotide construct including the SARS-Cov-2-specific double-stranded oligonucleotide according to Structural Formula (1) to Structural Formula (4), one to three phosphate groups may be bound at the 5' end of the antisense strand, and shRNA may be used instead of RNA, as will be apparent to those skilled in the art.

The hydrophilic material in Structural Formula (1) to Structural Formula (4) is preferably a polymer material having a molecular weight of 200 to 10,000, more preferably a polymer material having a molecular weight of 1,000 to 2,000. For example, the hydrophilic polymer material may be a nonionic hydrophilic polymer compound such as polyethylene glycol, polyvinylpyrrolidone, polyoxazoline, etc., but is not necessarily limited thereto.

In particular, the hydrophilic material (A) in Structural Formula (1) to Structural Formula (4) may be used in the form of a hydrophilic material block represented by Structural Formula (5) or Structural Formula (6) below. When these hydrophilic material blocks are used in an appropriate number (n in Structural Formula (5) or Structural Formula (6)) depending on the need, problems due to polydispersity that may occur in the case of using general synthetic polymer materials, etc. may be greatly alleviated.

Structural Formula (5) (A'ₘ-J)ₙ

Structural Formula (6) (J-A'ₘ)ₙ

In Structural Formula (5) or Structural Formula (6), A' is a hydrophilic material monomer, J is a linker connecting m hydrophilic material monomers to each other or connecting m hydrophilic material monomers and an oligonucleotide to each other, m is an integer of 1 to 15, n is an integer of 1 to 10, and the repeat unit represented by (A'ₘ-J) or (J-A'ₘ) is the basic unit of the hydrophilic material block.

When the hydrophilic material block represented by Structural Formula (5) or Structural Formula (6) is provided, the double-stranded oligonucleotide construct including the SARS-Cov-2-specific double-stranded oligonucleotide according to the present invention may have the structure of Structural Formula (7) or Structural Formula (8) below.

Structural Formula (7) (A'ₘ-J)ₙ-X-R-Y-B

Structural Formula (8) (J-A'ₘ)ₙ-X-R-Y-B

In Structural Formula (7) and Structural Formula (8), X, R, Y, and B are as defined in Structural Formula (1), and A', J, m, and n are as defined in Structural Formula (5) and Structural Formula (6).

In Structural Formula (5) to Structural Formula (8), any hydrophilic material monomer (A') may be used without limitation, so long as it meets the purpose of the present invention among the monomers of the nonionic hydrophilic polymer. Preferably, a monomer selected from among compound (1) to compound (3) shown in Table 1 below, more preferably a monomer of compound (1), is used, and G in compound (1) is preferably selected from among O, S, and NH.

In particular, among the hydrophilic material monomers, the monomer represented by compound (1) may be introduced with various functional groups, may exhibit superior biocompatibility such as having good in-vivo affinity and inducing less immune response, and may have the advantage of increasing the *in-vivo* stability of the double-stranded oligonucleotide contained in the construct according to Structural Formula (7) or Structural Formula (8) and increasing delivery efficiency, so it is very suitable for the production of the construct according to the present invention.

**[Table 1] Structure of hydrophilic material monomer in the present invention**

| Compound (1) | Compound (2) | Compound (3) |
|---|---|---|
| 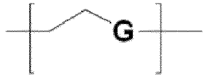 G being O, S, or NH | 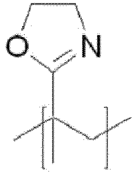 | 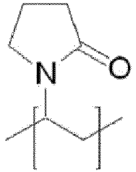 |

The hydrophilic material in Structural Formula (5) to Structural Formula (8) preferably has a total molecular weight in the range of 1,000 to 2,000.

For example, in Structural Formula (5) to Structural Formula (8), when hexaethylene glycol according to compound (1), namely a material in which G is O and m is 6, is used, the molecular weight of the hexaethylene glycol spacer is 344, and thus the number of repetitions (n) is preferably 3 to 5. In particular, the repeat units of the hydrophilic group represented by (A'ₘ-J)ₙ or (J-A'ₘ)ₙ in Structural Formula (5) to Structural Formula (8), namely the hydrophilic material blocks, may be used in an appropriate number represented by n, depending on the need. The hydrophilic material monomer A and the linker J included in each hydrophilic material block may be independently the same or different between hydrophilic material blocks. Specifically, when three hydrophilic material blocks are used (n=3), different hydrophilic material monomers may be used for respective hydrophilic material blocks, such as using the hydrophilic material monomer according to compound (1) for the first block, the hydrophilic material monomer according to compound (2) for the second block, and the hydrophilic material monomer according to compound (3) for the third block, or alternatively, any one hydrophilic material monomer selected from among the hydrophilic material monomers according to compounds (1) to (3) may be identically used for all of the hydrophilic material blocks. Likewise, as the linker that mediates binding of the hydrophilic material monomers, the same or different linkers may be used for hydrophilic material blocks. Also, m, which is the number of hydrophilic material monomers, may be the same or different between hydrophilic material blocks. Specifically, different numbers of hydrophilic material monomers may be used in a manner in which three hydrophilic material monomers are linked (m=3) in the first hydrophilic material block, five hydrophilic material monomers are linked (m=5) in the second hydrophilic material block, and four hydrophilic material monomers are linked (m=4) in the third hydrophilic material block, or alternatively, the same number of hydrophilic material monomers may be used for all of the hydrophilic material blocks.

Also, in the present invention, the linker (J) is preferably selected from the group consisting of -PO₃⁻-, - SO₃-, and -CO₂-, but is not limited thereto. Any linker may be used, so long as it meets the purpose of the present invention depending on the monomer of the hydrophilic material that is used, as will be apparent to those skilled in the art.

The hydrophobic material (B) in Structural Formula (1) to Structural Formula (4), Structural Formula (7), and Structural Formula (8) serves to form nanoparticles composed of the double-stranded oligonucleotide construct according to Structural Formula (1) to Structural Formula (4), Structural Formula (7), and Structural Formula (8) through hydrophobic interaction. The hydrophobic material preferably has a molecular weight of 250 to 1,000, and examples thereof may include, but are not limited to, a steroid derivative, glyceride derivative, glycerol ether, polypropylene glycol, C₁₂ to C₅₀ unsaturated or saturated hydrocarbon, diacyl phosphatidylcholine, fatty acid, phospholipid, lipopolyamine, etc. Any hydrophobic material may be used, so long as it meets the purpose of the present invention, as will be apparent to those skilled in the art.

The steroid derivative may be selected from the group consisting of cholesterol, cholestanol, cholic acid, cholesteryl formate, cholestanyl formate, and cholesteryl amine, and the glyceride derivative may be selected from among mono-, di-, and tri-glycerides. Here, the fatty acid of glyceride is preferably a C₁₂ to C₅₀ unsaturated or saturated fatty acid.

In particular, among examples of the hydrophobic material, a saturated or unsaturated hydrocarbon or cholesterol is preferable in that it facilitates binding in the step of synthesis of the double-stranded oligonucleotide construct according to the present invention, and a C₂₄ hydrocarbon, particularly a form including a disulfide bond, is most preferred.

The hydrophobic material is bound to the distal end of the hydrophilic material, and may be bound to any position of the sense strand or the antisense strand of siRNA.

The SARS-Cov-2-specific double-stranded oligonucleotide and the hydrophilic or hydrophobic material in Structural Formula (1) to Structural Formula (4), Structural Formula (7), and Structural Formula (8) according to the present invention are bound through a simple covalent bond or a linker-mediated covalent bond (X or Y). The linker mediating the covalent bond is covalently bound to the hydrophilic material or the hydrophobic material at the end of the SARS-Cov-2-specific double-stranded oligonucleotide, and is not particularly limited, so long as it provides a degradable bond in a certain environment, as necessary. Therefore, the linker may be any compound that binds to activate the SARS-Cov-2-specific double-stranded oligonucleotide and/or the hydrophilic material (or the hydrophobic material) during the production of the double-stranded oligonucleotide construct according to the present invention. The covalent bond may be either a non-degradable bond or a degradable bond. Here, the non-degradable bond includes an amide bond or a phosphate bond, and the degradable bond includes a disulfide bond, an acid-degradable bond, an ester bond, an anhydride bond, a biodegradable bond, or an enzyme-degradable bond, but the present invention is not limited thereto.

In addition, the SARS-Cov-2-specific double-stranded oligonucleotide represented by R (or S and AS) in Structural Formula (1) to Structural Formula (4), Structural Formula (7), and Structural Formula (8) may be used without limitation, so long as it is a double-stranded oligonucleotide capable of specifically binding to mRNA of SARS-Cov-2.

Preferably, in the present invention, it is composed of a sense strand including any one sequence selected from the group consisting of SEQ ID NOs: 1, 2, 3, 4, 5, 6, 7, 8, 9, and 10 and an antisense strand including a sequence complementary thereto.

In the double-stranded oligonucleotide construct including the SARS-Cov-2-specific double-stranded oligonucleotide according to the present invention, an amine group or a polyhistidine group may be additionally introduced to the end of the hydrophilic material opposite the end to which the oligonucleotide is bound.

This facilitates the intracellular introduction of a carrier and endosomal escape by the double-stranded oligonucleotide construct including the SARS-Cov-2-specific double-stranded oligonucleotide according to the present invention, and in order to facilitate the intracellular introduction of carriers such as quantum dots, dendrimers, and liposomes and endosomal escape, the introduction of an amine group and the use of a polyhistidine group and effects thereof have been reported.

Specifically, it is known that the primary amine group modified at the end or the outside of the carrier forms a conjugate through electrostatic interaction with a negatively charged gene while protonated at an in-vivo pH and also that the carrier may be protected from degradation of the lysosome because endosomal escape is facilitated due to the internal tertiary amine having a buffering effect at the low pH of the endosome after intracellular introduction (Gene transfer and expression inhibition using polymer-based hybrid material. Polymer Sci. Technol., Vol. 23, No.3, pp254-259).

It is known that histidine, which is a non-essential amino acid, has an imidazole ring (pK_{R} 6.04) at the residue (-R), and thus increases buffering capacity in endosomes and lysosomes, so histidine modification may be used to increase endosomal escape efficiency in non-viral gene carriers, including liposomes (Novel histidine-conjugated galactosylated cationic liposomes for efficient hepatocyte selective gene transfer in human hepatoma HepG2 cells. J. Controlled Release 118, pp262-270).

The amine group or polyhistidine group may be connected to the hydrophilic material or hydrophilic material block via at least one linker.

When an amine group or a polyhistidine group is introduced to the hydrophilic material of the double-stranded oligonucleotide construct according to Structural Formula (1) of the present invention, the structure represented by Structural Formula (9) below may be provided.

Structural Formula (9) P-J₁-J₂-A-X-R-Y-B

In Structural Formula (9), A, B, R, X, and Y are as defined in Structural Formula (1).

Also, P is an amine group or a polyhistidine group, and J₁ and J₂ are linkers. Here, J₁ and J₂ may be independently selected from among a simple covalent bond, PO₃⁻, SO₃, CO₂, C₂₋₁₂ alkyl, alkenyl, and alkynyl, but are not limited thereto, and any linker may be used as J₁ and J₂ meeting the purpose of the present invention depending on the hydrophilic material that is used, as will be apparent to those skilled in the art.

When an amine group is introduced, it is preferred that J₂ be a simple covalent bond or PO₃⁻ and that J₁ be a C₆ alkyl, but the present invention is not limited thereto.

Also, when a polyhistidine group is introduced, in Structural Formula (9), it is preferred that J₂ be a simple covalent bond or PO₃⁻ and that J₁ be compound (4), but the present invention is not limited thereto.

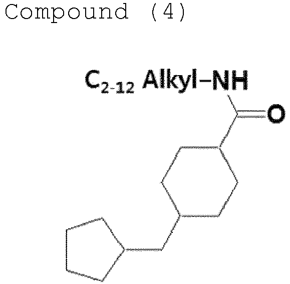

In addition, when the hydrophilic material of the double-stranded oligonucleotide construct according to Structural Formula (9) is a hydrophilic material block according to Structural Formula (5) or Structural Formula (6) and an amine group or polyhistidine group is introduced thereto, the structure represented by Structural Formula (10) or Structural Formula (11) below may be provided.

Structural Formula (10) P-J₁-J₂-(A'ₘ-J)ₙ-X-R-Y-B

Structural Formula (11) P-J₁-J₂-(J-A'ₘ)ₙ-X-R-Y-B

In Structural Formula (10) and Structural Formula (11), X, R, Y, B, A', J, m, and n are as defined in Structural Formula (7) or Structural Formula (8), and P, J₁, and J₂ are as defined in Structural Formula (9) .

In particular, in Structural Formula (10) and Structural Formula (11), the hydrophilic material is preferably in a form bound to the 3' end of the sense strand of the SARS-Cov-2-specific double-stranded oligonucleotide. Here, Structural Formula (9) to Structural Formula (11) may be provided in forms represented by Structural Formula (12) to Structural Formula (14) below.

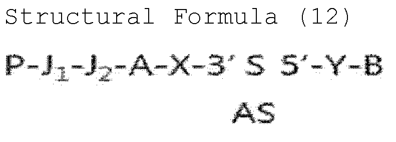

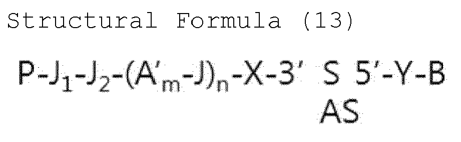

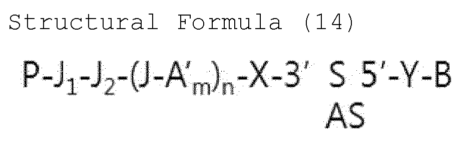

In Structural Formula (12) to Structural Formula (14), X, R, Y, B, A, A' J, m, n, P, J₁, and J₂ are as defined in Structural Formula (9) to Structural Formula (11), and 5' and 3' respectively represent the 5' end and the 3' end of the sense strand of the SARS-Cov-2-specific double-stranded oligonucleotide.

The amine group that may be introduced in the present invention may include primary to tertiary amine groups, particularly preferably a primary amine group. The amine group that is introduced may be provided in the form of an amine salt, and the salt of the primary amine group may be provided in the form of, for example, NH₃⁺.

In addition, the polyhistidine group that may be introduced in the present invention may include 3 to 10 histidines, preferably 5 to 8 histidines, most preferably 6 histidines. Additionally, at least one cysteine may be included, in addition to histidine.

Meanwhile, when a targeting moiety is provided to the double-stranded oligonucleotide construct including the SARS-Cov-2-specific double-stranded oligonucleotide according to the present invention and the nanoparticles formed therefrom, efficient delivery to a target cell may be promoted, and thus delivery thereof to a target cell is possible even at a relatively low dosage, thereby exhibiting a strong effect of regulating target gene expression and preventing a nonspecific SARS-Cov-2-specific double-stranded oligonucleotide from being delivered to other organs and cells.

Accordingly, the present invention provides a double-stranded oligo RNA construct in which a ligand (L), particularly a ligand that specifically binds to a receptor that promotes target-cell internalization through receptor-mediated endocytosis (RME), is additionally bound to the construct according to Structural Formula (1) to Structural Formula (4), Structural Formula (7), and Structural Formula (8). For example, the form in which a ligand is bound to the double-stranded oligo RNA construct according to Structural Formula (1) has the structure of Structural Formula (15) below.

Structural Formula (15) (Lᵢ-Z)-A-X-R-Y-B

In Structural Formula (15), A, B, X, and Y are as defined in Structural Formula (1), L is a ligand that specifically binds to a receptor that promotes target-cell internalization through receptor-mediated endocytosis (RME), and i is an integer of 1 to 5, preferably an integer of 1 to 3.

The ligand in Structural Formula (15) is preferably selected from among target-receptor-specific antibodies, aptamers, and peptides having RME properties that promote target-cell-specific cell internalization; and chemicals including folate (generally folate and folic acid are used interchangeably, and folate in the present invention refers to folate in a natural state or an activated state in the human body), hexosamines such as N-acetylgalactosamine (NAG), and sugars or carbohydrates such as glucose and mannose, but is not limited thereto.

Also, the hydrophilic material A in Structural Formula (15) may be used in the form of a hydrophilic material block according to Structural Formula (5) and Structural Formula (6).

Still another aspect of the present invention pertains to a method of producing a double-stranded oligonucleotide construct including a SARS-Cov-2-specific double-stranded oligonucleotide.

The process of producing the double-stranded oligonucleotide construct including the SARS-Cov-2-specific double-stranded oligonucleotide according to the present invention may include, for example:
(1) binding a hydrophilic material to a solid support;
(2) synthesizing a single-stranded oligonucleotide on the solid support to which the hydrophilic material is bound;
(3) covalently binding a hydrophobic material to the 5' end of the single-stranded oligonucleotide;
(4) synthesizing a single-stranded oligonucleotide having a sequence complementary to the sequence of the single-stranded oligonucleotide;
(5) separating and purifying an oligonucleotide/polymer construct and the single-stranded oligonucleotide from the solid support after completion of synthesis; and
(6) producing a double-stranded oligonucleotide construct through annealing of the oligonucleotide/polymer construct and the single-stranded oligonucleotide having the complementary sequence.

The solid support in the present invention is preferably controlled pore glass (CPG), but is not limited thereto, and polystyrene (PS), polymethyl methacrylate (PMMA) silica gel, cellulose paper, etc. may be used. CPG preferably has a diameter of 40 to 180 um and a pore size of 500 to 3000 Å. After step (5), the molecular weights of the purified RNA/polymer construct and single-stranded oligonucleotide are measured using a MALDI-TOF mass spectrometer to determine whether a desired oligonucleotide/polymer construct and single-stranded oligonucleotide are produced. In the production method described above, step (4) of synthesizing a single-stranded oligonucleotide having a sequence complementary to the sequence of the single-stranded oligonucleotide synthesized in step (2) may be performed before step (1) or during any one of steps (1) to (5).

Also, the single-stranded oligonucleotide having the sequence complementary to the sequence of the single-stranded oligonucleotide synthesized in step (2) may be used in a form in which a phosphate group is bound to the 5' end.

In addition, there is provided a method of producing a double-stranded oligonucleotide construct in which a ligand is additionally bound to the double-stranded oligonucleotide construct including the SARS-Cov-2-specific double-stranded oligonucleotide according to the present invention.

The method of producing the ligand-bound double-stranded oligonucleotide construct including the SARS-Cov-2-specific double-stranded oligonucleotide may include, for example:
(1) binding a hydrophilic material to a solid support to which a functional group is bound;
(2) synthesizing a single-stranded oligonucleotide on the solid support to which the functional group and the hydrophilic material are bound;
(3) covalently binding a hydrophobic material to the 5' end of the single-stranded oligonucleotide;
(4) synthesizing a single-stranded oligonucleotide having a sequence complementary to the sequence of the single-stranded oligonucleotide;
(5) separating a functional-group/oligonucleotide/polymer construct and the single-stranded oligonucleotide having the complementary sequence from the solid support after completion of synthesis;
(6) producing a ligand/oligonucleotide/polymer construct in the form of a single strand by binding a ligand to the end of the hydrophilic material using the functional group; and
(7) producing a ligand/double-stranded-oligonucleotide construct through annealing of the ligand/oligonucleotide/polymer construct and the single-stranded oligonucleotide having the complementary sequence.

After step (6), the produced ligand/oligonucleotide/polymer construct and the single-stranded oligonucleotide having the complementary sequence are separated and purified, and the molecular weights thereof are then measured using a MALDI-TOF mass spectrometer to determine whether a desired ligand/oligonucleotide/polymer construct and complementary oligonucleotide are produced. The ligand/double-stranded oligonucleotide construct may be produced through annealing of the ligand/oligonucleotide/polymer construct and the single-stranded oligonucleotide having the complementary sequence. In the production method described above, step (4) of synthesizing a single-stranded oligonucleotide having the sequence complementary to the sequence of the single-stranded oligonucleotide synthesized in step (3) may be an independent synthesis process, and may be performed before step (1) or during any one of steps (1) to (6).

Yet another aspect of the present invention pertains to nanoparticles including the double-stranded oligonucleotide construct according to the present invention. The double-stranded oligonucleotide construct according to the present invention forms self-assembled nanoparticles through hydrophobic interaction of the hydrophobic material (Korean Patent No. 1224828). These nanoparticles not only have vastly superior delivery efficiency into the body and in-vivo stability, but also have excellent particle size uniformity, which facilitates quality control (QC), thereby simplifying the process of manufacture into a drug.

In the present invention, the nanoparticles may be configured such that one or more double-stranded oligonucleotide constructs, preferably two or more double-stranded oligonucleotide constructs, including different double-stranded oligonucleotides, are mixed. In one embodiment, the nanoparticles may include one type of SARS-Cov-2-specific double-stranded oligonucleotide including a sense strand including any one sequence selected from among SEQ ID NOs: 1, 2, 3, 4, 5, 6, 7, 8, 9, and 10 and an antisense strand including a sequence complementary thereto, and in another embodiment, the nanoparticles may include different types of SARS-Cov-2-specific double-stranded oligonucleotides including a sense strand including any one sequence selected from among SEQ ID NOs: 1, 2, 3, 4, 5, 6, 7, 8, 9, and 10 and an antisense strand including a sequence complementary thereto, and may also include a SARS-Cov-2-specific double-stranded oligonucleotide not disclosed in the present invention.

Still yet another aspect of the present invention pertains to a pharmaceutical composition for treating COVID-19 containing the double-stranded oligonucleotide, the double-stranded oligonucleotide construct, or the nanoparticles according to the present invention as an active ingredient.

The composition of the present invention may be prepared by including at least one pharmaceutically acceptable carrier in addition to the active ingredient described above for administration. The pharmaceutically acceptable carrier must be compatible with the active ingredient of the present invention, and may include saline, sterile water, Ringer's solution, buffered saline, dextrose solution, maltodextrin solution, glycerol, and ethanol, which may be used alone or in combination of two or more thereof. Other typical additives such as antioxidants, buffers, and bacteriostats may be added as necessary. Also, diluents, dispersants, surfactants, binders, and lubricants may be additionally added to form an injectable formulation such as an aqueous solution, suspension, emulsion, and the like. In particular, it is preferable to provide a lyophilized formulation. For the preparation of the lyophilized formulation, a method commonly known in the art to which the present invention belongs may be used, and a stabilizer for lyophilization may be added. Preferably, it is possible to prepare a formulation depending on each disease or component using an appropriate method in the art or a method disclosed in Remington's Pharmaceutical Science (Mack Publishing Company, Easton PA).

The type of composition of the present invention may be determined by those skilled in the art based on the symptoms of a typical patient and the severity of the disease. In addition, it may be formulated in various forms such as powders, tablets, capsules, solutions, injections, ointments, syrups, and the like, and may be provided in unit-dose or multiple-dose containers, for example, sealed ampoules and bottles.

The composition of the present invention may be administered orally or parenterally. The route of administration of the composition according to the present invention is not limited thereto, and for example, oral, inhalational, intravenous, intramuscular, intraarterial, intramedullary, intradural, intracardiac, transdermal, subcutaneous, intraperitoneal, enteral, sublingual, or topical administration is possible.

In particular, since COVID-19 is a respiratory disease, intranasal administration, preferably intranasal administration using a spray method, or administration to the lungs through intratracheal instillation is possible, but the present invention is not limited thereto.

The dosage of the composition according to the present invention may vary depending on the patient's body weight, age, gender, health status, diet, administration time, method, excretion rate, severity of disease, etc., and may be easily decided by those skilled in the art. Moreover, the composition of the present invention may be formulated into a suitable dosage form for clinical administration using known techniques.

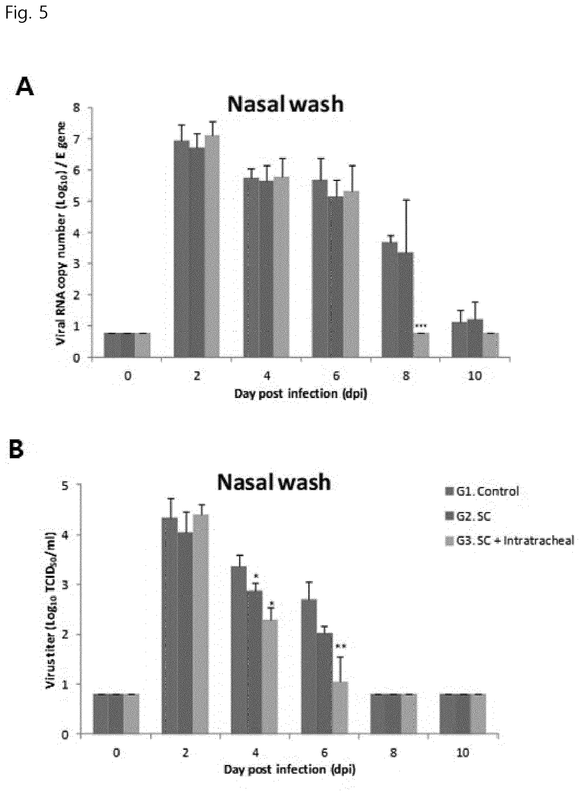

In an embodiment of the present invention, ferrets were used, and specifically, SAMiRNA^{™} targeting the SARS-CoV-2 sequence according to the present invention was administered to ferrets infected with SARS-CoV-2, and the SARS-CoV-2 copy number was measured through qRT-PCR in the nasal wash of ferrets, confirming that the number of SARS-CoV-2 viruses was significantly reduced in the subcutaneous injection + intratracheal administration group compared to the infection control group, and also viral titer was measured using TCID50, confirming that the viral titer was statistically significantly decreased in both the subcutaneous injection group and the subcutaneous injection + intratracheal administration group compared to the infection control group (FIG. 5).

Even still yet another aspect of the present invention pertains to a lyophilized formulation including the pharmaceutical composition according to the present invention.

A further aspect of the present invention pertains to a method of treating COVID-19 including administering the pharmaceutical composition according to the present invention to a subject in need of treatment for COVID-19.

Still a further aspect of the present invention pertains to the use of the double-stranded oligonucleotide, the double-stranded oligonucleotide construct including the double-stranded oligonucleotide, and the nanoparticles including the same for the treatment of COVID-19.

Yet a further aspect of the present invention pertains to the use of the pharmaceutical composition for the treatment of COVID-19.

Still yet a further aspect of the present invention pertains to the use of the double-stranded oligonucleotide, the double-stranded oligonucleotide construct including the double-stranded oligonucleotide, and the nanoparticles including the same for the manufacture of a medicament for the treatment of COVID-19.

### [Advantageous Effects]

According to the present invention, a double-stranded oligonucleotide construct including a SARS-Cov-2-specific double-stranded oligonucleotide and a pharmaceutical composition containing the same as an active ingredient can inhibit the expression of SARS-Cov-2 with high efficiency without side effects, and are very effective at treating COVID-19.

### [Description of Drawings]

FIG. 1 shows a process of selecting candidate sequences each composed of 19 nucleotides by applying a 1-base sliding-window algorithm to a genome in order to design SARS-CoV-2-specific oligonucleotide candidate sequences;
FIGs. 2A and 2B show results of measurement of E and Rdrp gene Ct values after infecting a SAMiRNA-treated Huh7 cell line with COVID-19;
FIG. 3 shows a process for evaluating the *in-vivo* antiviral efficacy of SAMiRNA-COVID19;
FIG. 4 shows changes in body weight and body temperature of ferrets in the evaluation of the *in-vivo* antiviral efficacy of SAMiRNA-COVID19; and
FIG. 5 shows results of measurement of SARS-CoV-2 viral RNA copy number and viral titer in the nasal wash sample of ferrets in the evaluation of the *in-vivo* antiviral efficacy of SAMiRNA-COVID19.

### [Mode for Invention]

A better understanding of the present invention may be obtained through the following examples. These examples are merely set forth to illustrate the present invention and are not construed as limiting the scope of the present invention, as will be apparent to those skilled in the art. Therefore, the substantial scope of the present invention will be defined by the appended claims and equivalents thereto.

### Example 1. Algorithm for screening of oligonucleotide targeting SARS-CoV-2 and selection of candidate sequences

siRNA-based drug high-throughput screening is a method for generating all possible candidate sequences by applying a sliding-window algorithm to an entire RNA virus genome sequence, removing unnecessary candidate sequences through homology filtering, and determining the extent of inhibition of virus replication by all of the finally selected oligonucleotides.

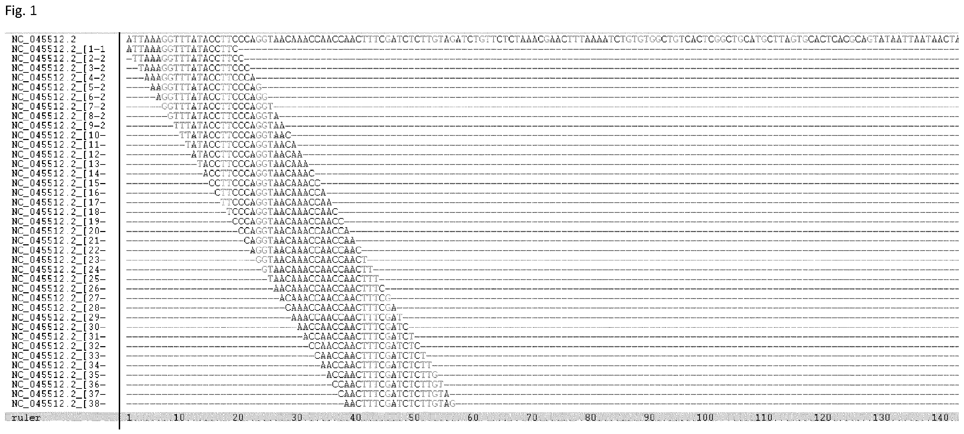

A design process for oligonucleotide candidate sequences for SARS-CoV-2 was performed in a manner in which a 1-base sliding-window algorithm was applied to the SARS-CoV-2 reference genome (NC_045512.2, 29,903 bp) to thus generate 29,885 candidate sequences each composed of 19 nucleotides (FIG. 1).

The generated oligonucleotide candidate sequence list was performed with a BLAST e-value of 100 or less for human total reference seq RNA, and as such, 6,885 candidate sequences having identities of 15 nucleotides or less to human genes and RNA sequences were selected. Thereamong, 560 candidate sequences having all 19 nucleotides identical to SARS-CoV (NC_004718.3, 29,751 bp) were selected. In consideration of siRNA efficiency, 480 candidate sequences with a GC content of 30 to 60 were finally selected.

### Example 2. Synthesis of double-stranded oligo RNA construct

The double-stranded oligo RNA construct (SAMiRNA) produced in the present invention has a structure according to the following structural formula.

C₂₄-5' S 3'-(hexaethylene glycol-PO₃⁻)₃-hexaethylene glycol AS 5'-PO₄

The sense strand of a monoSAMiRNA (n=4) double-stranded oligo construct was synthesized as follows. Specifically, three dimethoxytrityl (DMT) hexaethylene glycol phosphoramidates, which are hydrophilic material monomers, were successively bound through the above reaction using 3,4,6-triacetyl-1-hexa(ethylene glycol)-N-acetyl galactosamine-CPG as a support, RNA or DNA synthesis was performed, and then C₂₄(C₆-S-S-C₁₈) containing a disulfide bond, which is a hydrophobic material, was additionally bound to the 5' end, thereby synthesizing the sense strand of monoSAMiRNA (n=4) in which NAG-hexaethylene glycol-(-PO₃⁻ hexaethylene glycol)₃ was bound to the 3' end and C₂₄(C₆-S-S-C₁₈) was bound to the 5' end.

After completion of synthesis, the synthesized single-stranded RNA and oligo (DNA or RNA)/polymer construct were separated from CPG using 28% (v/v) ammonia in a water bath at 60°C, followed by a deprotection reaction to remove protective residues. After removal of the protective residues, the single-stranded RNA and the oligo (DNA or RNA)/polymer construct were treated with N-methylpyrrolidone, triethylamine, and triethylamine trihydrofluoride at a volume ratio of 10:3:4 in an oven at 70°C to remove 2'TBDMS (tert-butyldimethylsilyl). The single-stranded RNA, the oligo (DNA or RNA)/polymer construct, and the ligand-bound oligo (DNA or RNA)/polymer construct were separated from the reaction products through high-performance liquid chromatography (HPLC), and the molecular weights thereof were measured using a MALDI-TOF mass spectrometer (MALDI TOF-MS, SHIMADZU, Japan) to determine whether they matched the nucleotide sequence and the oligo/polymer construct to be synthesized. Thereafter, in order to produce each double-stranded oligo construct, the sense strand and the antisense strand were mixed in equal amounts, added to a 1X annealing buffer (containing 30 mM HEPES, 100 mM potassium acetate, and 2 mM magnesium acetate, pH 7.0-7.5), allowed to react in a constant-temperature water bath at 90°C for 3 minutes, and further allowed to react at 37°C, thereby producing desired SAMiRNA. Annealing of the double-stranded oligo RNA constructs thus produced was confirmed through electrophoresis.

### Example 3. High-throughput screening of RNAi-inducing SAMiRNA nanoparticles targeting SARS-CoV-2

### 3.1 Preparation of SAMiRNA nanoparticles

960 SAMiRNAs targeting the SARS-CoV-2 sequence synthesized in Example 2 were dissolved in a 1X Dulbecco's phosphate-buffered saline (DPBS) (WELGENE, KR), filtered with a 96-well Filtration Plate MultiScreen_{HTS} GV Filter Plate, 0.22 µm, clear, sterile (MERCK, DE), and then used in the experiment for the present invention.

### 3.2 Intracellular processing of SAMiRNA nanoparticles

In order to discover SAMiRNA that inhibits the expression of SARS-CoV-2, Huh7, which is a human-derived liver cancer cell line, was used, and Huh-7 cells (JCRB, JP) were seeded in a 96-well plate 24 hours before treatment with SAMiRNA. RPMI 1640 medium (sh30027.01) (Hyclone, US) was used as a cell culture medium, and 100 µl thereof was added to each well, followed by cell culture at 37°C and 5% CO₂. The RPMI 1640 medium was supplemented with 2.05 mM L-glutamine, 1% penicillin-streptomycin (Hyclone, US), and 100 fetal bovine serum (Hyclone, US). The cells were treated once with SAMiRNA candidates at a concentration of 10 µM with two medium replacements, followed by culture at 37°C and 5% CO₂ for 24 hours.

### 3.3 Intracellular SARS-CoV-2 infection

After removing the medium from the 96-well cell culture plate cultured in Example 3.2, each well was treated with the prepared virus at an MOI of 0.01 and infected for 1 hour and 30 minutes. Thereafter, the medium was replaced with an RPMI 1640 medium (virus growth medium) containing 2% fetal bovine serum, followed by cell culture at 37°C and 5% CO₂ for 2 days. Finally, the cell culture fluid was harvested from the cell culture plate and then mixed with Cartridge No. 1 (Lysis buffer) and Proteinase K of the Exiprep^{™} 96 Viral DNA/RNA Kit (BIONEER, KR), followed by culture at 65°C for 2 hours.

### 3.4 SAMiRNA screening through analysis of SARS-CoV-2 gRNA expression inhibitory efficacy

In order to extract gRNA from the cell culture fluid obtained in Example 3.3, ExiPrep^{™} 96 Lite (BIONEER, KR) and ExiPrep^{™} 96 Viral DNA/RNA Kit (BIONEER, KR), which are automated nucleic acid extraction instruments, were used. The extracted nucleic acids were analyzed for E and Rdrp genes using the Exicycler^{™} 96 Real-Time Quantitative Thermal Block (BIONEER, KR) according to the manufacturer's protocol of AccuPower^{®} SARS-CoV-2 Real-Time RT-PCR Kit (BIONEER, KR). Here, RT-qPCR was performed 45 cycles of 30 minutes at 50°C, 10 minutes at 94°C, 15 seconds at 95°C, and 1 minute at 60°C.

All Ct values derived from biological/technical repeats of the E and Rdrp genes obtained from the virus-infected cell culture samples treated with SAMiRNA were determined. Based on the above results, the optimal SAMiRNA candidates for each gene region of SARS-CoV-2 were selected. The selected SAMiRNA candidates were compared with 7259 sequences registered in the COVID-19 database and tested for gene sequence variation.

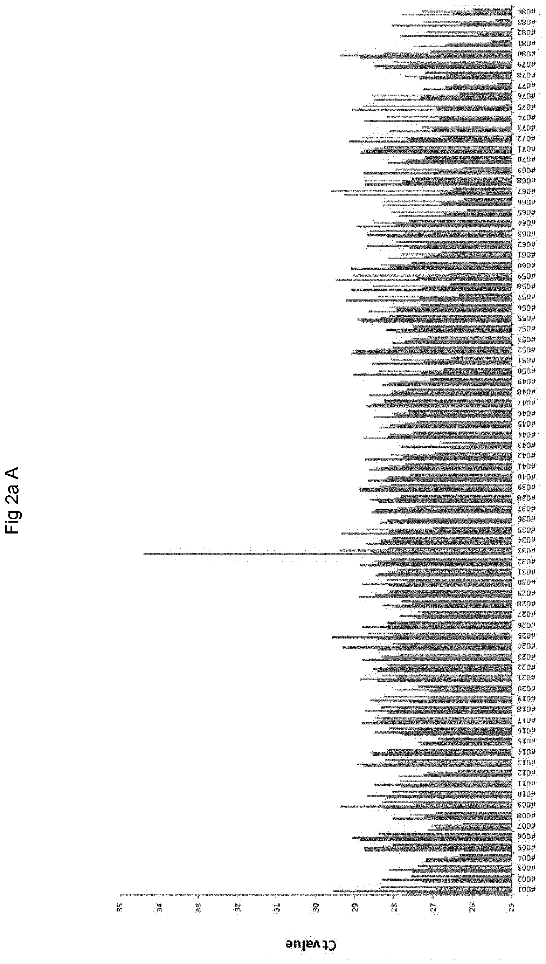

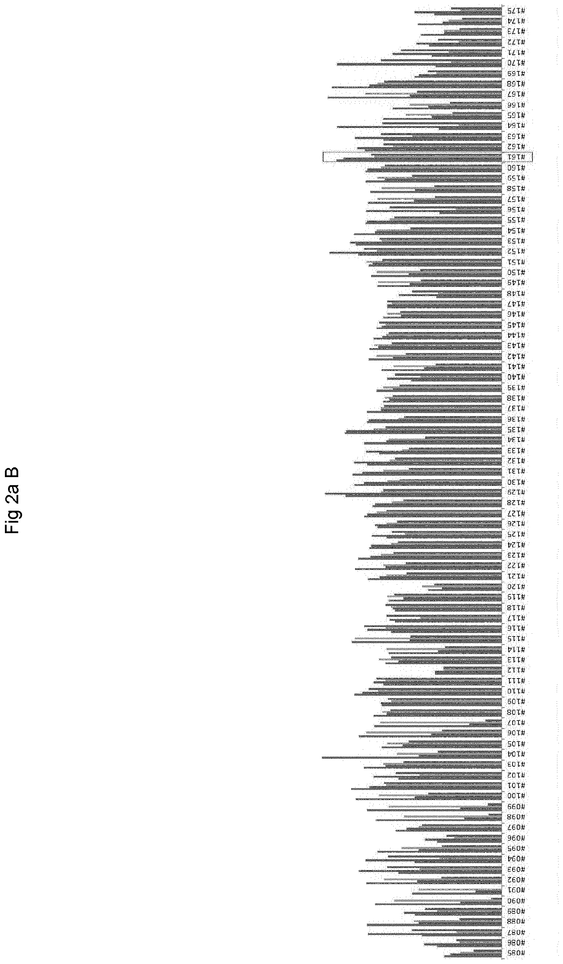

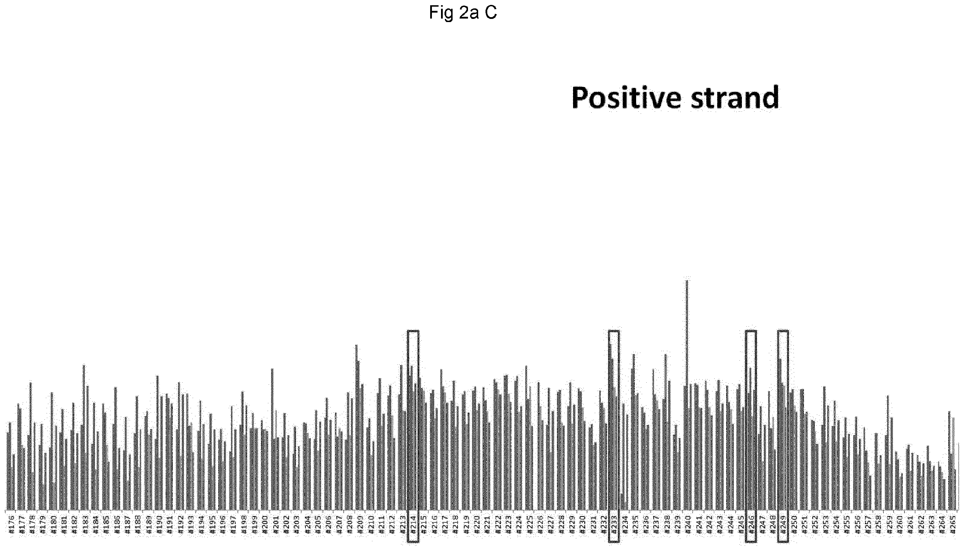

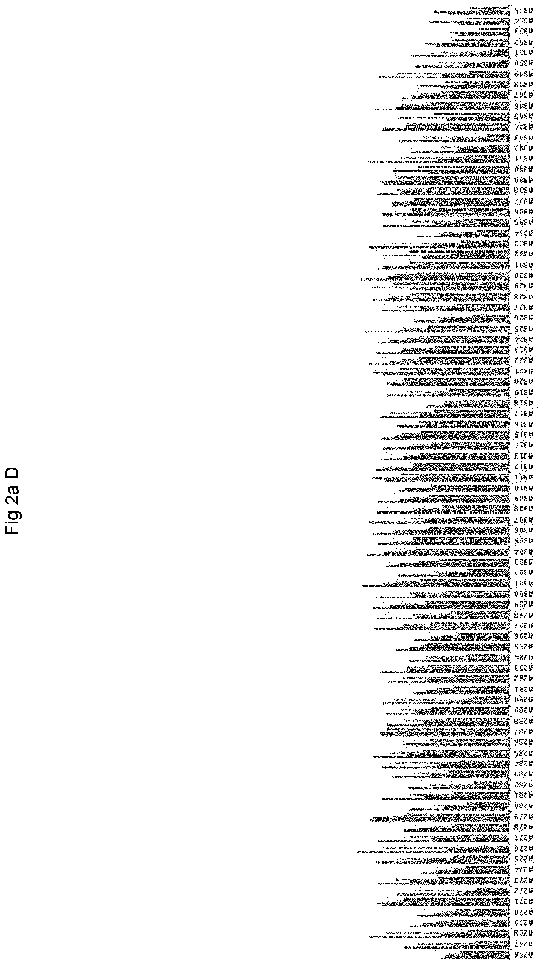

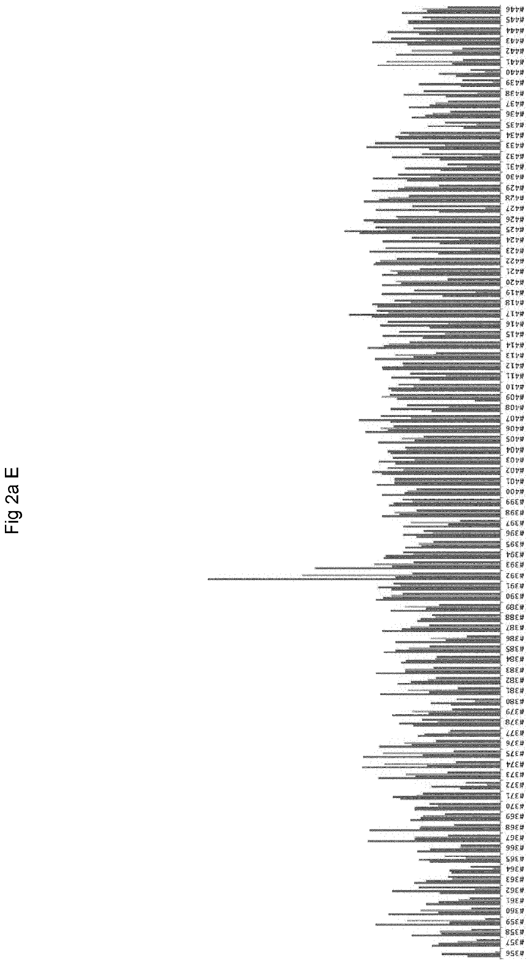

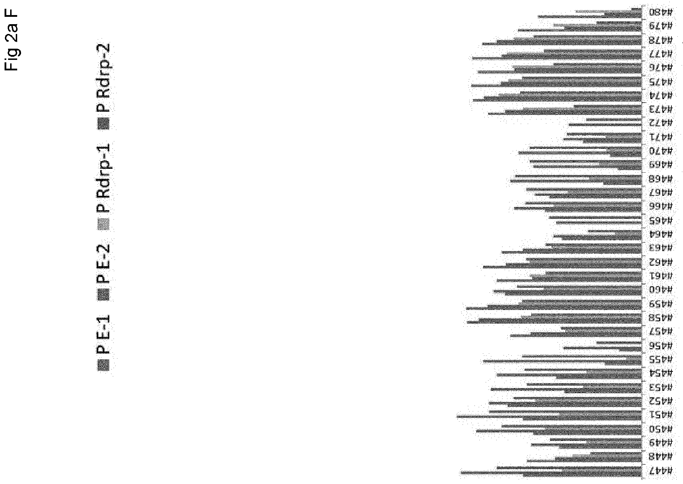

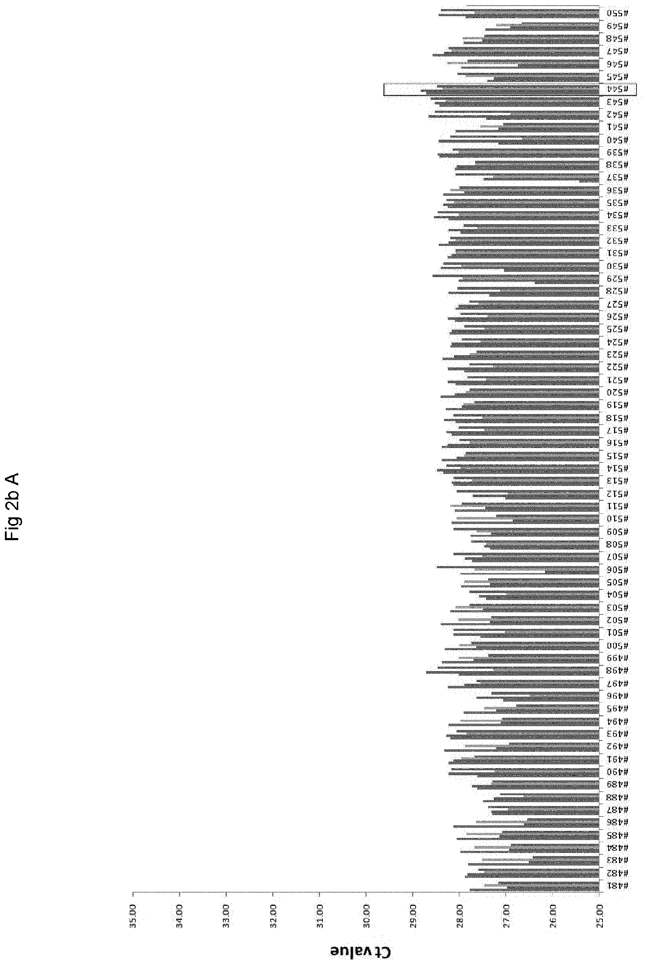

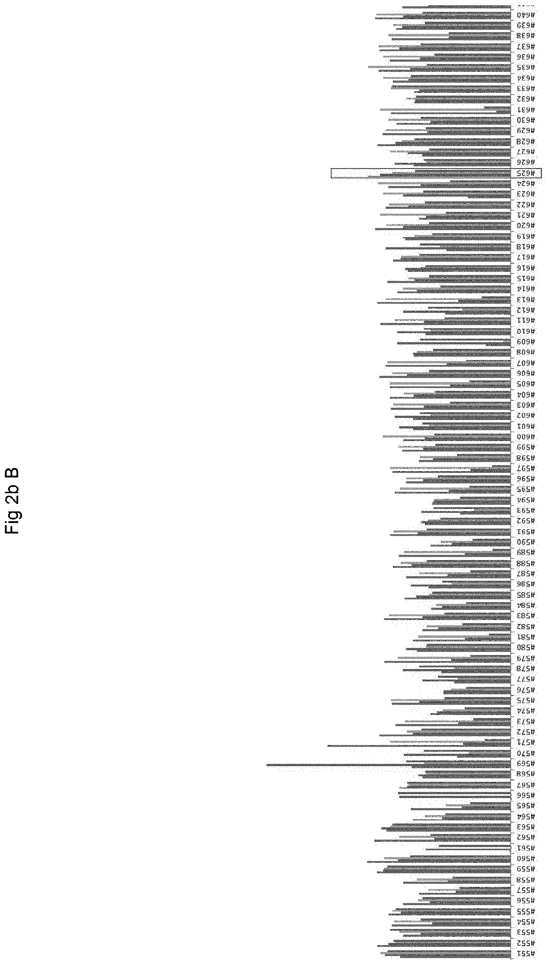

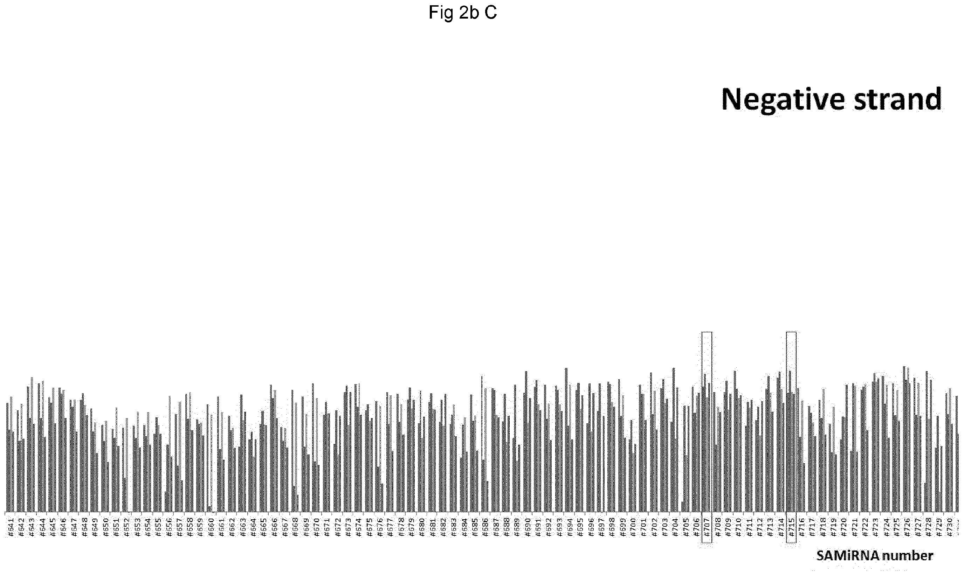

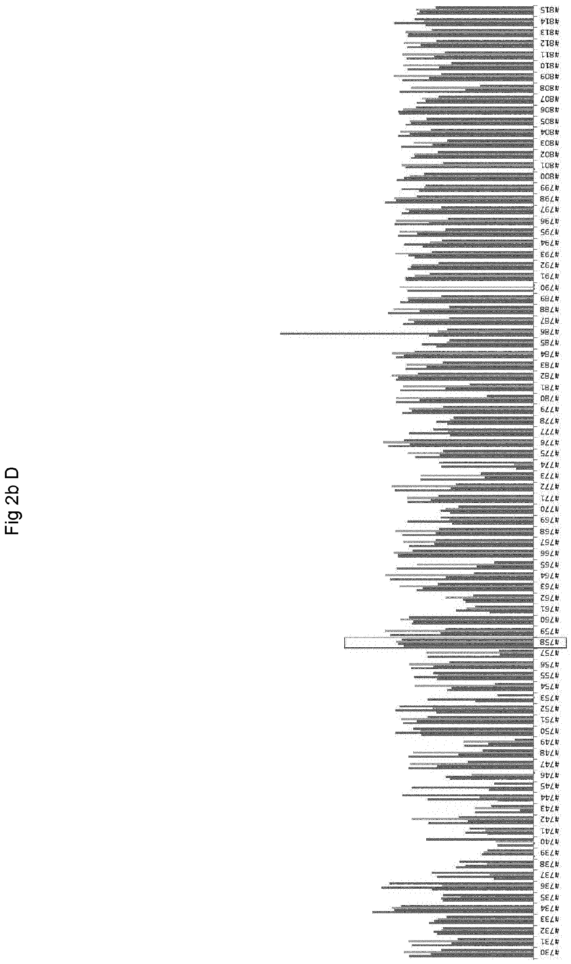

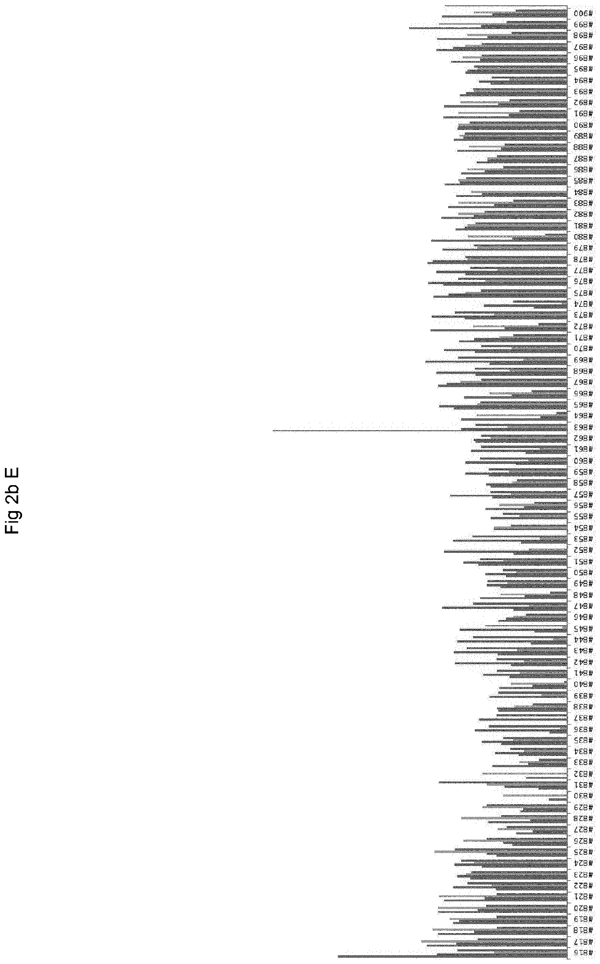

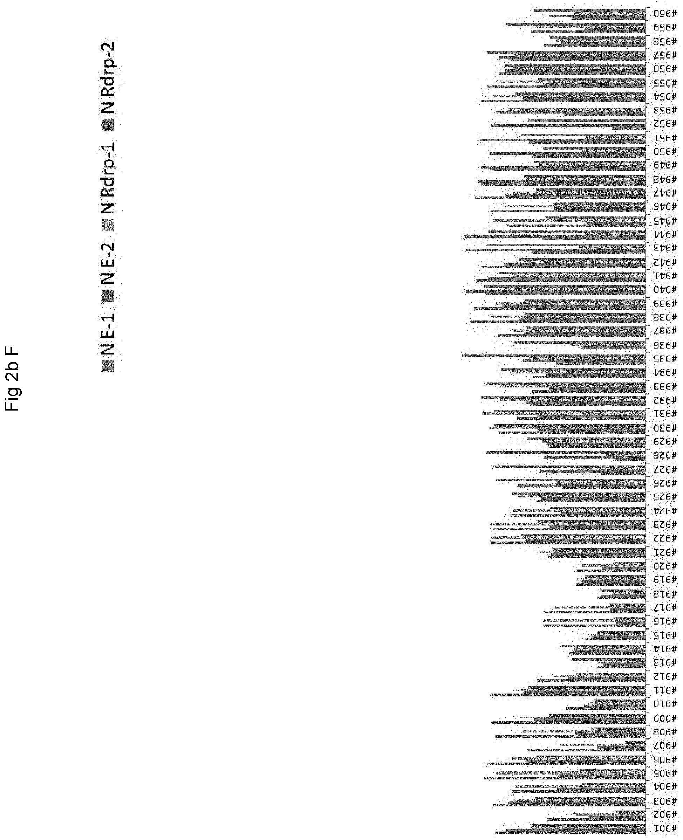

Consequently, as shown in Table 2 below, 10 types of SAMiRNA having SARS-CoV-2 inhibitory efficacy were finally selected. The selected SAMiRNA candidates are SAMiRNAs having the sequences of SEQ ID NOs: 1, 2, 3, 4, 5, 6, 7, 8, 9, and 10, respectively, in the order described. The selected SEQ ID NOs: 1, 2, 3, 4, 5, 6, 7, 8, 9, and 10 correspond to #161, #214, #233, #246, #249, #544, #625, #707, #715, and #758 of FIG. 2, respectively.

**[Table 2]**

| SARS-CoV-2-specific oligonucleotide candidates selected through 1-base sliding-window screening | | | | | | |
|---|---|---|---|---|---|---|
| **SEQ ID NO:** | **Accession No.** | **Position** | **Sense strand sequence** | **SEQ ID NO:** | **Antisense strand sequence** | **Identity** |
| 1 | NC_045512.2 | 15094-15112 | AATAGAGCTCGCACCGTAG | 11 | CUACGGUGCGAGCUCUAUU | 99.99% |
| 2 | NC_045512.2 | 17088-17106 | TGGTACTGGTAAGAGTCAT | 12 | AUGACUCUUACCAGUACCA | 99.83% |
| 3 | NC_045512.2 | 18255-18273 | GTTTATCACCCGCGAAGAA | 13 | UUCUUCGCGGGUGAUAAAC | 99.92% |
| 4 | NC_045512.2 | 20457-20475 | CATAACAGATGCGCAAACA | 14 | UGUUUGCGCAUCUGUUAUG | 99.89% |
| 5 | NC_045512.2 | 20776-20794 | ATAATGATGAATGTCGCAA | 15 | UUGC GACAUUCAUCAUUAU | 99.96% |
| 6 | NC_045512.2 | 10488-10506 | TTAAAACCAACACTACCAC | 16 | GUGGUAGUGUUGGUUUUAA | 100.00% |
| 7 | NC_045512.2 | 15038-15056 | ATAGTAGGGATGACATTAC | 17 | GUAAUGUCAUCCCUACUAU | 100.00% |
| 8 | NC_045512.2 | 17957-17975 | TCTCTATCAGACATTATGC | 18 | GCAUAAUGUCUGAUAGAGA | 100.00% |
| 9 | NC_045512.2 | 18257-18275 | GCTTCTTCGCGGGTGATAA | 19 | UUAUCACCCGCGAAGAAGC | 99.94% |
| 10 | NC_045512.2 | 25509-25527 | CCATCCGAAAGGGAGTGAG | 20 | CUCACUCCCUUUCGGAUGG | 99.71% |

### Example 4. Evaluation of symptom relief efficacy of SAMiRNA^{™} in SARS-CoV-2-infected ferret model

### 4.1 Preparation of SAMiRNA^{™} nanoparticles

A mixture of 10 types of SAMiRNA^{™} targeting the SARS-CoV-2 sequence shown in Table 2 was dissolved in a 1X Dulbecco's phosphate-buffered saline (DPBS) (WELGENE, KR), filtered with a 96-well Filtration Plate MultiScreen_{HTS} GV Filter Plate, 0.22 µm, clear, sterile (MERCK, DE), and then used in the experiment for the present invention.

### 4.2 SARS-CoV-2-infected ferret animal test method

A total of 11 female ferrets with an average age of 15 months (IDBio, Korea) were divided into a control group untreated after infection including 3 ferrets, a subcutaneous injection (sc) group including 4 ferrets, and a subcutaneous injection + intratracheal administration group including 4 ferrets.

0.5 mL of SARS-CoV-2 (CBNU-nCoV-01) was inoculated at 1×10^{5.5} TCID50/mL into each of the nasal cavity and bronchi of ferrets. 24 hours after virus infection, SAMiRNA^{™} nanoparticles were administered once to each experimental animal through subcutaneous injection (50 mpk) and through subcutaneous injection (50 mpk) + intratracheal administration (500 µl).

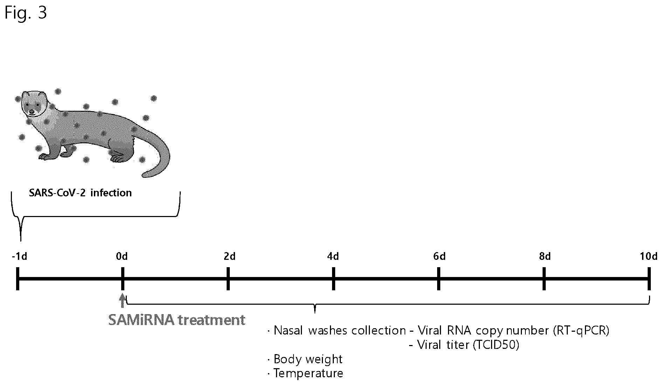

Changes in body weight and body temperature of individual subjects in the control and test groups were measured on day 0 before virus infection and on days 2, 4, 6, 8, and 10 after infection, and nasal wash samples were collected (FIG. 3). The collected samples were subjected to viral titer measurement using a TCID50 method, or RNA was extracted therefrom and then absolute quantitative analysis of viral RNA copy number was performed through qRT-PCR according to the manufacturer's protocol of AccuPower^{®} SARS-CoV-2 Real-Time RT-PCR Kit (BIONEER, KR).

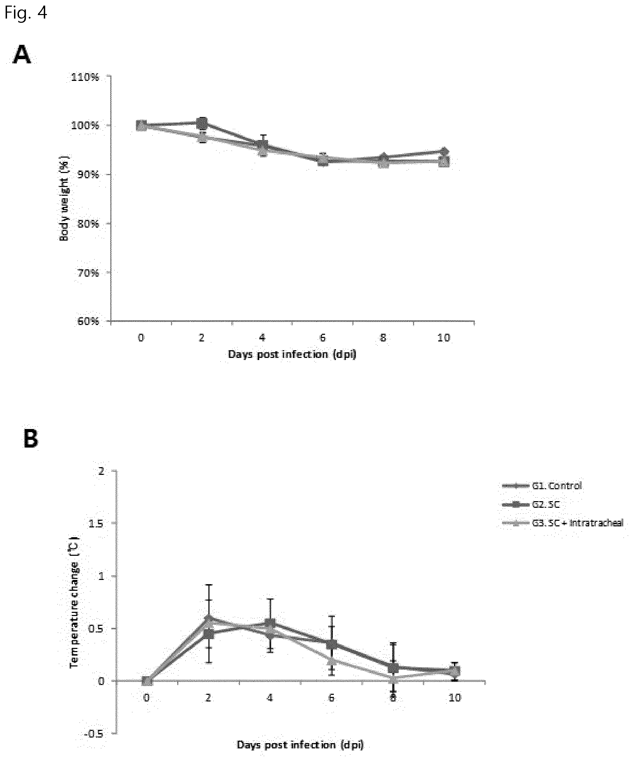

Consequently, the body weight of ferrets after virus infection was reduced by 7-8% compared to before infection, and body temperature was returned to normal after an average increase of 0.5°C or higher at 4 dpi, and there was no significant difference by SAMiRNA treatment (FIG. 4).

Based on the results of measurement of the SARS-CoV-2 copy number through qRT-PCR in the nasal wash of ferrets, the number of SARS-CoV-2 viruses was significantly reduced in the subcutaneous injection + intratracheal administration group at 8 dpi compared to the infection control group (FIG. 5A) .

Based on the results of measurement of the viral titer using TCID50, the viral titer was statistically significantly reduced in both the subcutaneous injection group and the subcutaneous injection + intratracheal administration group at 4 dpi compared to the infection control group. Statistical significance was confirmed in the subcutaneous injection + intratracheal administration group even at 6 dpi compared to the infection control group (FIG. 5B).

Although specific embodiments of the present invention have been disclosed in detail above, it will be obvious to those skilled in the art that the description is merely of preferable exemplary embodiments and is not to be construed as limiting the scope of the present invention. Therefore, the substantial scope of the present invention will be defined by the appended claims and equivalents thereto. Simple modifications or changes of the present invention can be easily used by those of ordinary skill in the art, and all such modifications or changes can be considered to be included in the scope of the present invention.

## Claims

1. A double-stranded oligonucleotide specific to SARS-Cov-2, comprising a sense strand comprising any one sequence selected from the group consisting of SEQ ID NOs: 1, 2, 3, 4, 5, 6, 7, 8, 9, and 10 and an antisense strand comprising a sequence complementary thereto.

2. The double-stranded oligonucleotide according to claim 1, wherein the sense strand or the antisense strand comprises 19 to 31 nucleotides.

3. The double-stranded oligonucleotide according to claim 1, wherein the oligonucleotide is siRNA, shRNA, or miRNA.

4. The double-stranded oligonucleotide according to claim 1, wherein each of the sense strand and the antisense strand is independently DNA or RNA.

5. The double-stranded oligonucleotide according to claim 1, wherein the sense strand or the antisense strand of the double-stranded oligonucleotide comprises a chemical modification.

6. The double-stranded oligonucleotide according to claim 5, wherein the chemical modification is at least one selected from the group consisting of:
a modification in which a hydroxyl group (-OH) at a 2' carbon position of a sugar structure in a nucleotide is substituted with any one selected from the group consisting of methyl (-CH₃), methoxy (-OCH₃), amine (-NH₂), fluorine (-F), O-2-methoxyethyl, O-propyl, O-2-methylthioethyl, O-3-aminopropyl, O-3-dimethylaminopropyl, O-N-methylacetamido, and O-dimethylamidooxyethyl;
a modification in which oxygen of the sugar structure in the nucleotide is substituted with sulfur;
a modification in which a nucleotide bond is any one bond selected from the group consisting of a phosphorothioate bond, a boranophosphate bond, and a methyl phosphonate bond; and
a modification into PNA (peptide nucleic acid), LNA (locked nucleic acid), or UNA (unlocked nucleic acid).

7. The double-stranded oligonucleotide according to claim 1, wherein at least one phosphate group is bound to a 5' end of the antisense strand of the double-stranded oligonucleotide.

8. A double-stranded oligonucleotide construct specific to SARS-Cov-2, having a structure of Structural Formula (1) below:
Structural Formula (1) A-X-R-Y-B
in Structural Formula (1), A is a hydrophilic material, B is a hydrophobic material, X and Y are each independently a simple covalent bond or a linker-mediated covalent bond, and R is the double-stranded oligonucleotide according to any one of claims 1 to 7.

9. The double-stranded oligonucleotide construct according to claim 8, having a structure of Structural Formula (2) below: 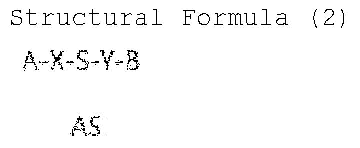 in Structural Formula (2), S and AS respectively represent a sense strand and an antisense strand of the double-stranded oligonucleotide according to claim 8, and A, B, X, and Y are as defined in claim 8.

10. The double-stranded oligonucleotide construct according to claim 9, having a structure of Structural Formula (3) or Structural Formula (4) below: 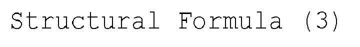 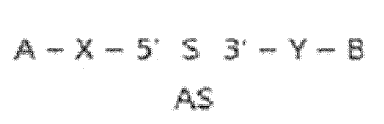 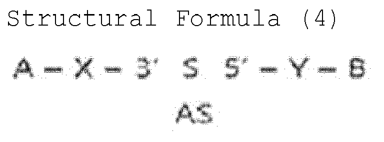 in Structural Formulas (3) and (4), A, B, X, Y, S, and AS are as defined in claim 9, and 5' and 3' respectively represent a 5' end and a 3' end of the sense strand of the double-stranded oligonucleotide.

11. The double-stranded oligonucleotide construct according to claim 8, wherein the hydrophilic material is selected from the group consisting of polyethylene glycol (PEG), polyvinylpyrrolidone, and polyoxazoline.

12. The double-stranded oligonucleotide construct according to claim 8, wherein the hydrophilic material has a structure of Structural Formula (5) or Structural Formula (6) below:
Structural Formula (5) **(A'ₘ-J)ₙ**
Structural Formula (6) **(J-A'ₘ)ₙ**
in Structural Formula (5) or Structural Formula (6), A' is a hydrophilic material monomer, J is a linker connecting m hydrophilic material monomers to each other or connecting m hydrophilic material monomers and a double-stranded oligonucleotide to each other, m is an integer of 1 to 15, n is an integer of 1 to 10, the hydrophilic material monomer A' is any one compound selected from among compounds (1) to (3) below, and the linker (J) is selected from the group consisting of -PO₃⁻-, -SO₃-, and -CO₂-: 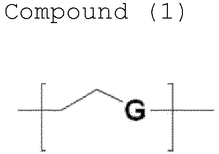
in compound (1), G is selected from the group consisting of O, S, and NH; 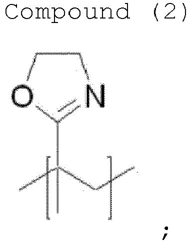 and

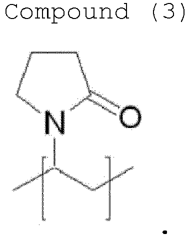

13. The double-stranded oligonucleotide construct according to claim 12, having a structure of Structural Formula (7) or Structural Formula (8) below:
Structural Formula (7) (A'ₘ-J)ₙ-X-R-Y-B
Structural Formula (8) (J-A'ₘ)ₙ-X-R-Y-B.

14. The double-stranded oligonucleotide construct according to claim 8, wherein the hydrophilic material has a molecular weight of 200 to 10,000.

15. The double-stranded oligonucleotide construct according to claim 8, wherein the hydrophobic material has a molecular weight of 250 to 1,000.

16. The double-stranded oligonucleotide construct according to claim 15, wherein the hydrophobic material is any one selected from the group consisting of a steroid derivative, glyceride derivative, glycerol ether, polypropylene glycol, C₁₂ to C₅₀ unsaturated or saturated hydrocarbon, diacyl phosphatidylcholine, fatty acid, phospholipid, lipopolyamine, lipid, tocopherol, and tocotrienol.

17. The double-stranded oligonucleotide construct according to claim 16, wherein the steroid derivative is any one selected from the group consisting of cholesterol, cholestanol, cholic acid, cholesteryl formate, cholestanyl formate, and cholesteryl amine.

18. The double-stranded oligonucleotide construct according to claim 16, wherein the glyceride derivative is any one selected from the group consisting of monoglycerides, diglycerides, and triglycerides.

19. The double-stranded oligonucleotide construct according to claim 8, wherein the covalent bond represented by X and Y is a non-degradable bond or a degradable bond.

20. The double-stranded oligonucleotide construct according to claim 19, wherein the non-degradable bond is an amide bond or a phosphate bond.

21. The double-stranded oligonucleotide construct according to claim 19, wherein the degradable bond is any one selected from the group consisting of a disulfide bond, an acid-degradable bond, an ester bond, an anhydride bond, a biodegradable bond, and an enzyme-degradable bond.

22. Nanoparticles comprising the double-stranded oligonucleotide construct according to any one of claims 8 to 21.

23. The nanoparticles according to claim 22, wherein the nanoparticles are configured such that at least two double-stranded oligonucleotide constructs comprising different double-stranded oligonucleotides are mixed.

24. A pharmaceutical composition for treating COVID-19 comprising the double-stranded oligonucleotide according to any one of claims 1 to 7 or the double-stranded oligonucleotide construct according to any one of claims 9 to 21 as an active ingredient.

25. A pharmaceutical composition for treating COVID-19 comprising the nanoparticles according to claim 22 as an active ingredient.

26. A lyophilized formulation comprising the pharmaceutical composition according to claim 24.

27. A lyophilized formulation comprising the pharmaceutical composition according to claim 25.
